# EUROPÄISCHE PATENTSCHRIFT

(11) **EP 1 359 140 B1**
(45) Veröffentlichungstag und Bekanntmachung des Hinweises auf die Patenterteilung: **29.10.2008**
(21) Anmeldenummer: 03008667.2
(22) Anmeldetag: 16.04.2003
(51) Int. Cl.: C07C 229/24, C07C 227/08, A01N 37/44

(54) **Herstellung und Verwendung von Iminodibernsteinsäureammoniummetallsalzen**
Process and use of iminodisuccinic acid ammonium metal salts
Procédé et utilisation de sels métalliques d'ammonium d'acide iminosuccinique

(30) Priorität: 29.04.2002 DE 10219037
(43) Veröffentlichungstag der Anmeldung: 05.11.2003
(73) Patentinhaber: LANXESS Deutschland GmbH, 51369 Leverkusen (DE)
(72) Erfinder: Groth, Torsten, Dr., 51519 Odenthal (DE); Mitschker, Alfred, Dr., 51519 Odenthal (DE); Moritz, Ralf-Johann, Dr., 41469 Neuss (DE); Klein, Thomas, Dr., 50678 Köln (DE); Menzel, Thomas, Dr., 40723 Hilden (DE); Wirth, Wolfgang, Dr., 51429 Bergisch Gladbach (DE)

(56) Entgegenhaltungen:
- WO-A-98/45251
- US-A1- 2001 044 381
- DATABASE CA [Online] CHEMICAL ABSTRACTS SERVICE, COLUMBUS, OHIO, US; ANZAI, RYUICHI ET AL: "Antimicrobial mixture compositions" retrieved from STN Database accession no. 135:328372 CA XP002245341 & JP 2001 302415 A (MITSUBISHI RAYON CO., LTD., JAPAN) 31. Oktober 2001 (2001-10-31)
- DATABASE CA [Online] CHEMICAL ABSTRACTS SERVICE, COLUMBUS, OHIO, US; MAEKAWA, KAZUO ET AL: "Fatty acid soap-based powdered detergent compositions" retrieved from STN Database accession no. 133:311163 CA XP002245342 & JP 2000 290698 A (ASAHI DENKA KOGYO K. K., JAPAN;COOP CLEAN K. K.) 17. Oktober 2000 (2000-10-17)
- DATABASE CA [Online] CHEMICAL ABSTRACTS SERVICE, COLUMBUS, OHIO, US; ASAKAWA, YOSHIAKI ET AL: "Water-soluble detergent builder with improved biodegradability and detergent compositions" retrieved from STN Database accession no. 124:320186 CA XP002245343 & JP 08 041490 A (NIPPON CATALYTIC CHEM IND, JAPAN) 13. Februar 1996 (1996-02-13)

## Beschreibung

Die Erfindung betrifft ein Verfahren zur Herstellung von Iminodibernsteinsäureammoniummetallsalzen durch Umsetzung von Maleinsäureanhydrid, Alkalimetallhydroxiden, Ammoniak und Wasser in einer ersten Stufe zu Iminodibernsteinsäureammoniumsalzen und deren anschließende Umsetzung mit Metalloxiden, Metallhydroxiden oder anderen Metallsalzen in einer zweiten Stufe. Die daraus resultierenden Produkte können zur Erhöhung der Verfügbarkeit von Metallionen z.B. in der Landwirtschaft als Spurennährstoffdünger oder Schneckenkorn oder in der Keramikindustrie zur Färbung von Oberflächen eingesetzt werden.

Spurennährstoffdünger werden in der Landwirtschaft zur Steigerung der landwirtschaftlichen Erträge und zur Verhinderung von Pflanzenkrankheiten eingesetzt. Dabei werden z.B. spezielle Calcium-, Magnesium-, Mangan-, Eisen-, Kupfer- oder Zinksalze von Ethylendiamintetraessigsäure (EDTA), Diethylentriaminpentaessigsäure (DTPA) oder anderen Komplexiermitteln verwendet. Pflanzenkrankheiten wie die Apfelstippe (braune Flecken) oder die Fruchtendfäule der Tomate (schwarze Flecken) sind auf eine gestörte Versorgung mit Calcium zurückzuführen.

EDTA und DTPA sind zwei klassische Komplexiermittel, die seit Jahren in großen Mengen auch in anderen Verwendungsbereichen eingesetzt werden. Viele der klassischen Komplexiermittel, wie EDTA und DTPA und verschiedene Phosphonate und die daraus resultierenden Metallkomplexe sind nicht oder nur bedingt biologisch abbaubar, remobilisieren Schwermetalle in Oberflächengewässern und können sogar bis in die Trinkwasseraufbereitung gelangen, da sie weder in Klärschlämmen noch in Böden adsorbiert werden.

Daher ist es eine wichtige Aufgabe, Komplexiermittel zu entwickeln, die die bisherigen ökotoxikologischen Nachteile nicht aufweisen. Iminodibernsteinsäure ist nun ein Komplexiermittel, das eine leichte biologische Abbaubarkeit zeigt und damit gegenüber den bisherigen Komplexiermittel einen ökotoxikologischen Vorteil besitzt. Von zusätzlichem Vorteil ist, dass auch seine Metallsalze, die in der Landwirtschaft und in der Keramikindustrie verwendet werden können, biologisch abbaubar sind. Ein besonderer Vorteil ist, dass auch das Herstellungsverfahren umweltfreundlich ist, da es keine nennenswerten Abfälle oder Abwässer erzeugt und eventuell freigesetzter Ammoniak in chemische Prozesse zurückgeführt werden kann.

Für Iminodibernsteinsäure sind auf der Basis von Maleinsäureanhydrid oder Maleinsäure und Ammoniak bisher folgende Herstellungsmöglichkeiten bekannt: In GB 1 306 331 wird die Herstellung von Iminodibernsteinsäure aus Maleinsäure und Ammoniak im Molverhältnis von 2:3 bis 2:5 bei Temperaturen von 60 bis 155°C beschrieben. Zur Aufarbeitung werden entweder Salzsäure oder Natronlauge hinzugegeben. In SU 0 639 863 wird Iminodibernsteinsäure in Gegenwart von Alkalimetallhydroxiden aus Maleinsäure und Ammoniak bei einem Molverhältnis von 2:0,8 bis 2:1 und Temperaturen von 110 bis 130°C hergestellt. In JP 6/329 606 wird ein dreistufiges Verfahren zur Herstellung von Iminodibernsteinsäure beschrieben. Zunächst reagiert ein Maleinsäurederivat mit Ammoniak in wässrigem Medium. Danach erfolgt die Zugabe von Alkali- oder Erdalkalimetallhydroxiden. In der dritten Verfahrensstufe schließt sich ein sogenannter Reifungsprozess an. In JP 6/329 607 wird ebenfalls ein dreistufiges Verfahren zur Herstellung von Iminodibernsteinsäure beschrieben. In der ersten Stufe wird wiederum zunächst ein Maleinsäurederivat mit Ammoniak in wässrigem Medium umgesetzt. Danach erfolgt in der zweiten Stufe der Zusatz von Alkali- oder Erdalkalimetallhydroxiden. In der dritten Stufe wird nach Zusatz von weiterem Maleinsäurederivat die Reaktion fortgesetzt.

In keinem der Patente wird ein Verfahren zur Herstellung und die Verwendung der erfindungsgemäßen Iminodibernsteinsäureammoniummetallsalze beschrieben.

Aus US-A 6 107 518 ist ein Verfahren zur Herstellung von Iminodisuccinatalkalimetallsalzen sowie deren Eignung als Komplexbildner bekannt. Aus US 2001/0 044 381 A1 ist bekannt, dass Komplexe mit Calcium, Magnesium, Barium, Strontium, Mangan, Zink, Kupfer und Eisen hergestellt werden können. Keines der Dokumente beschreibt jedoch ein gezieltes Verfahren zur Herstellung der erfindungsgemäßen Iminodibernsteinsäureammoniummetallsalze worin nicht alle Ammoniumreste gegen Alkali-, Erdalkali- oder Schwermetalle ausgetauscht werden.

Die vorliegende Erfindung betrifft deshalb ein Verfahren zur Herstellung von Iminodibernsteinsäureammoniummetallsalzen, dadurch gekennzeichnet, dass in einer ersten Stufe Maleinsäureanhydrid (MSA), Alkalimetallhydroxid und Wasser im Molverhältnis von 2 : 0 - 3 : 5 - 30 vermischt werden und dann Ammoniak im Verhältnis MSA : Ammoniak 2 : 1,5 - 8 hinzudosiert wird um Iminodibernsteinsäureammoniumsalze der Formel 6
IDS(NH₄)ₓ(Na)_{y}(K)_{z}(H)ₘ Formel 6
worin IDS für den Iminodibernsteinsäurerest steht,
mit x = 0,1 - 4, y = 0 - 3, z = 0 - 3 und m = 0 - 2
zu erhalten, die in einer zweiten Stufe mit Metalloxiden, Metallhydroxiden oder anderen Metallsalzen zu Iminodibernsteinsäureammoniummetallsalzen der Formel 8 umgesetzt werden,
IDS (NH₄)ₓ (Na)_{y} (K)_{z} (Me)ₘ • (NH₃)ₙ Formel 8
in der
IDS für den Iminodibernsteinsäurerest steht und
x = 0,1 - 3,9
y = 0 - 3
z = 0 - 3
m = 0,1 - 2
n = 0 - 6
bedeutet und

Me für Metalle der II., III. und IV. Hauptgruppe, der I. bis VIII. Nebengruppe sowie für Metalle der Lanthaniden des Periodensystems steht, die in den Oxidationsstufen 1, 2, 3 oder 4 vorkommen können.

Im erfindungsgemäßen Verfahren werden in der ersten Stufe Wasser, Maleinsäureanhydrid (MSA), Alkalimetallhydroxid (AlkaliOH) und Ammoniak (NH₃) in einen Reaktor eindosiert und das entstandene Maleinsäuresalz bei den genannten Reaktionstemperaturen (T) und Reaktionszeiten (t) umgesetzt. Das erhaltene Reaktionsgemisch wird dann in der zweiten Stufe, gegebenenfalls nach Zugabe von Wasser und Abdestillation von Ammoniakwasser, wobei hochkonzentrierte, lagerstabile und geruchsarme Iminodibernsteinsäureammoniumsalzlösungen hergestellt werden können, mit Metalloxiden, Metallhydroxiden oder anderen Metallsalzen unter den genannten Reaktionstemperaturen (T) und Reaktionszeiten (t) zu Iminodibemsteinsäureammoniummetallsalzen umgesetzt. Unter Zugabe von Wasser und Abdestillation von Ammoniakwasser werden gegebenenfalls nach einer Klärfiltration hochkonzentrierte, lagerstabile und geruchsarme Produktlösungen hergestellt. Die darin enthaltenen Salze können auch durch Trocknung in Feststoffe überführt werden.

Das erfindungsgemäße Verfahren hat den Vorteil, dass es sowohl diskontinuierlich als auch kontinuierlich durchgeführt und dabei ein hohes Maß an Wirtschaftlichkeit erreicht werden kann. Dieser Sachverhalt ist von großer Bedeutung, da auch umweltfreundliche Produkte trotz aller Vorteile nur konkurrenzfähig sind, wenn sie unter entsprechend ökonomischen Bedingungen hergestellt werden können. Das erfindungsgemäße Verfahren erzeugt nahezu keinen Abfall, da nach der Destillation des Ammoniaks dieser recycliert oder in anderen Prozessen verwertet werden kann und das verbleibende Produkt vollständig verwendet wird. Lediglich bei der Klärfiltration können geringe Mengen an abfiltriertem Material entstehen. Die erfindungsgemäßen Produkte sind darüber hinaus biologisch abbaubar. Im Verfahren und Produkt werden Ökonomie und Ökologie in sehr effizienter Weise miteinander vereint.

Im erfindungsgemäßen Verfahren werden in der ersten Stufe zunächst MSA, Alkalimetallhydroxid und Wasser im Molverhältnis von MSA : AlkaliOH : Wasser = 2 : 0 - 3 : 5 - 30 miteinander vermischt. Die Vermischung der Komponenten kann sowohl in einer kontinuierlichen als auch in einer diskontinuierlichen Prozessführung erfolgen. Dabei werden MSA und Alkalimetallhydroxid entweder simultan, nacheinander oder abwechselnd in Portionen in das Wasser gegeben. In einer bevorzugten Ausführungsform liegt der pH-Wert bei dieser Dosierung bei < 11, besonders bevorzugt bei < 8. Die Dosierung erfolgt bevorzugt bei Temperaturen von 50 - 150°C, besonders bevorzugt bei 70 - 120°C. Die Dosierzeit ist abhängig von der Ausführungsform. Es entstehen Maleinsäurealkalimetallsalze in Lösung oder Suspension, bevorzugt in Lösung, besonders bevorzugt in konzentrierter Lösung mit Feststoffgehalten von über 30 Gew.-%, bevorzugt über 40 Gew.-%, besonders bevorzugt über 50 Gew.-%. Nach der beendeten Dosierung können die Lösungen oder Suspensionen nachgerührt werden.

In einer besonderen Ausführungsform beträgt das Molverhältnis von MSA : AlkaliOH = 2 : 0. Dabei entstehen alkalifreie Maleinsäurelösungen. In einer weiteren besonderen Ausführungsform beträgt das Molverhältnis von MSA : AlkaliOH = 2 : 0,2 - 2,8, bevorzugt 2 : 0,5 - 2,5, besonders bevorzugt 2 : 1,5 - 2,3. In bevorzugter Weise beträgt das Molverhältnis von MSA : Wasser = 2 : 5,5 - 25, besonders bevorzugt 2 : 6 - 20.

Im Falle einer sachgemäßen Durchführung werden nur geringe Mengen an Nebenkomponenten wie zum Beispiel Fumarsäure und Äpfelsäure bzw. deren Salze gebildet. Daher werden Maleinsäure bzw. dessen Salze mit Ausbeuten von über 90 %, bevorzugt über 95 %, besonders bevorzugt über 98 % erhalten.

Im Hinblick auf eine kontinuierliche Prozessführung hat sich die kontinuierliche und simultane Dosierung von MSA-Schmelze und Alkalimetallhydroxidlösungen in eine vorgelegte, Maleinsäuresalzlösung als besonders vorteilhaft erwiesen. Auf diese Weise können sogar sehr reine und auch farblose Lösungen mit ebenso hohen Ausbeuten erhalten werden.

Zu den im Rahmen der ersten Stufe gebildeten Maleinsäure- bzw. Maleinsäuresalz-haltigen Suspensionen oder Lösungen wird Ammoniak dosiert. Das Molverhältnis von MSA : Ammoniak beträgt 2 : 1,5 - 8, bevorzugt 2 : 1,6 - 6, besonders bevorzugt 2 : 1,8 - 5. Die Zugabe kann gleichermaßen sowohl in kontinuierlicher als auch in diskontinuierlicher Prozessführung erfolgen.

Im Verfahren kann MSA in Form von Schmelze, Schuppen oder Briketts, bevorzugt als Schmelze eingesetzt werden. Alkalimetallhydroxide werden in Substanz oder in wässriger Lösung, z.B. in Konzentrationen von 10 - 60 Gew.-%, bevorzugt von 20 - 55 Gew.-% und besonders bevorzugt von 25 - 50 Gew.-% verwendet. Als Alkalimetallhydroxide können Lithiumhydroxid, Natriumhydroxid oder Kaliumhydroxid und bevorzugt Natriumhydroxid oder Kaliumhydroxid ausgewählt werden. Ammoniak kann flüssig, gasförmig oder als wässrige Lösung eindosiert werden.

Die aus MSA, AlkaliOH, Ammoniak und Wasser gebildeten Maleinsäureammoniumsalzlösungen werden bei Temperaturen von 70 - 170°C, bevorzugt bei 75 - 160°C, besonders bevorzugt bei 80 - 150°C, ganz besonders bevorzugt bei 85 - 140°C und Reaktionszeiten von 0,1 - 100 h, bevorzugt 0,5 - 70 h, besonders bevorzugt 1 - 50 h, ganz besonders bevorzugt 2 - 40 h umgesetzt. Die Reaktion kann sowohl in Konti- als auch in Diskonti-Reaktoren durchgeführt werden. Bei der Reaktionstemperatur können in einem Prozess ein oder mehrere Temperaturniveaus eingestellt werden.

Die Reaktion wird in der Regel unter dem sich automatisch einstellenden Druck durchgeführt. Dabei können Drücke von bis zu 50 bar, bevorzugt bis zu 30 bar, besonders bevorzugt bis zu 20 bar auftreten. Eine Überlagerung mit Inertgasen, besonders in Diskonti-Reaktoren, kann dabei zusätzlich vorgenommen werden, wobei Drücke bis zu 80 bar zulässig sind.

In einer besonderen Ausführungsförm können überraschenderweise alkalifreie Maleinsäureammoniumsalzlösungen in offenen Reaktoren bei Normaldruck umgesetzt werden, ohne dass nennenswerte Mengen an Ammoniak entweichen.

Durch die Reaktionsbedingungen wird ein Maleinsäure-Umsatz von >93 %, bevorzugt >95 %, besonders bevorzugt >98 % des theoretischen Umsatzes erreicht.

In der ersten Stufe des erfindungsgemäßen Verfahrens werden so Iminodibernsteinsäureammoniumsalzlösungen erhalten, die entweder direkt in der zweiten Stufe zur Bildung der Iminodibernsteinsäureammoniummetallsalze eingesetzt werden, oder die gegebenenfalls erst nach einer Aufarbeitung zur Herstellung der Iminodibernsteinsäureammoniummetallsalze eingesetzt werden.

Der Aufarbeitungsschritt ist von erheblichem Vorteil, wenn die Herstellung der Iminodibernsteinsäureammoniummetallsalze zeitlich nicht unmittelbar im Anschluss oder räumlich nicht in der gleichen Produktionsstätte erfolgen kann. Durch die Aufarbeitung, in der durch eine Zugabe von Wasser und die Abdestillation von Ammoniakwasser der Feststoffgehalt und der Ammoniak- bzw. Ammoniumgehalt verringert wird, gelingt es hochkonzentrierte, lagerstabile und geruchsarme Iminodibemsteinsäureammoniumsalzlösungen herzustellen. Dadurch wird eine für die heutige Zeit unerlässliche, flexible Folgeproduktion ermöglicht.

In einer bevorzugten Ausführungsform werden daher die nach der Reaktion erhaltenen Umsetzungsgemische zunächst mit Wasser und gegebenenfalls mit Alkalimetallhydroxid versetzt und durch die Abdestillation von Ammoniakwasser, die pH-Einstellung mit Ammoniak oder Ammoniakwasser, die Konzentrationseinstellung mit Wasser und gegebenenfalls durch eine Klärfiltration in hochkonzentrierte, lagerstabile, schwach gefärbte und geruchsarme Iminodibernsteinsäureammoniumsalzlösungen umgewandelt.

Das Molverhältnis von ursprünglich eingesetztem MSA : AlkaliOH beträgt 2 : 0 - 3, bevorzugt 2 : 0 - 2. Die Abdestillation des Ammoniakwassers erfolgt bei Temperaturen von 50 - 150°C, bevorzugt bei 60 - 130°C, besonders bevorzugt bei 70 - 110°C, ganz besonders bevorzugt bei 75 - 105°C und Drücken von 0,1 - 10 bar, bevorzugt 0,2 - 2 bar innerhalb von 0,1 - 50 h, bevorzugt 0,3 - 30 h, besonders bevorzugt 0,5 - 25 h, ganz besonders bevorzugt 0,9 - 20 h.

Die Aufarbeitung kann wiederum als Bestandteil eines kontinuierlichen oder diskontinuierlichen Gesamtprozesses durchgeführt werden. Besonders vorteilhaft hat sich für die Abdestillation des Ammoniakwassers die Einspeisung von Wasserdampf erwiesen. Die Feststoffgehalte betragen nach der Aufarbeitung über 20 Gew.-%, bevorzugt über 30 Gew.-%, besonders bevorzugt über 40 Gew.-%. Die Lösungen sind unter den für Transport und Lagerung üblichen Temperaturbedingungen z.B. von 0 - 50°C, bevorzugt 1 - 40°C, besonders bevorzugt von 2 - 35°C lagerstabil.

Die in der ersten Stufe erfindungsgemäß hergestellten Iminodibernsteinsäureammoniumsalzlösungen zeigen folgende Zusammensetzung: Iminodibernsteinsäure und ihre Salze (Formel 1) als Gemisch der Stereoisomere ( S,S-IDS, R,R-IDS und R,S-IDS ) in Ausbeuten von >65 %, bevorzugt >70 %, besonders bevorzugt >74 % der theoretischen Ausbeute. Die Summe aller Nebenkomponenten und ihrer Salze liegen in Mengen von <35 %, bevorzugt <30 %, besonders bevorzugt <26 % der theoretischen Mengen vor, wobei Maleinsäure und ihre Salze (Formel 2) mit <7 %, bevorzugt <5 %, besonders bevorzugt <2 % der theoretischen Menge, Fumarsäure und ihre Salze (Formel 3) mit <20 %, bevorzugt <15 %, besonders bevorzugt <10 % der theoretischen Menge, Äpfelsäure als Gemisch der Stereoisomere ( R- und S-Äpfelsäure ) und ihre Salze (Formel 4) mit <7 %, bevorzugt <5 %, besonders bevorzugt <3 % der theoretischen Menge und Asparaginsäure als Gemisch der Stereoisomere ( R- und S-Asparaginsäure ) und ihre Salze (Formel 5) mit <25 %, bevorzugt <20 %, besonders bevorzugt <15 % der theoretischen Menge vorliegen. 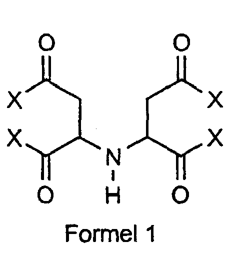 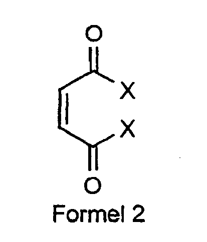 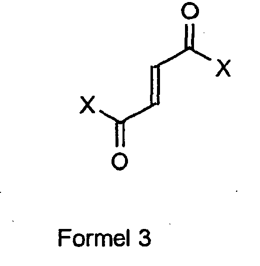 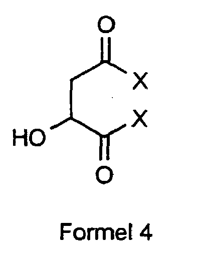 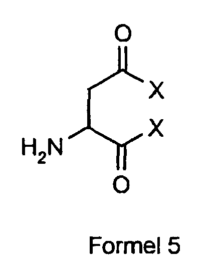 X = OH, OLi, ONa, OK, ONH₄

Insgesamt werden Produktlösungen erhalten, in denen die angegebenen Komponenten der Formeln 1-5 in Summenausbeuten von >93 %, bevorzugt >96 %, besonders bevorzugt >98 % der theoretischen Ausbeute vorliegen. Der Bioabbau der Produkte liegt entsprechend dem OECD 301 E-Test nach 28d über 70 %, meist über 72 %, vielfach über 74 %. So wird z.B. das IDS(NH₄)₃-Salz bereits nach 28 d zu 90 % abgebaut. Im OECD 302 B-Test werden das IDSK₂NH₄-Salz nach bereits 14 d zu 99 % und das IDS(NH₄)₃-Salz nach 14 d zu 97 % abgebaut.

Erfindungsgemäß liegen in Abhängigkeit von der eingesetzten Ammoniakmenge die Carboxylgruppen der Iminodibernsteinsäure und ihrer Nebenkomponenten besonders bevorzugt in der Ammoniumsalzform vor. Je nach Menge des in der ersten Stufe eingesetzten Alkalimetallhydroxids erhält man beispielsweise Iminodibernsteinsäureammoniumsalze mit Natrium-, Lithium- oder Kalium-Anteilen. Die erfindungsgemäß bevorzugten Zwischenprodukte der ersten Stufe sind Iminodibernsteinsäureammoniumsalze der Formel 1 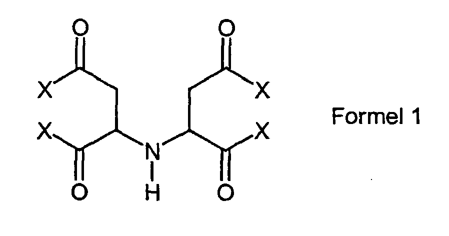 worin X für OLi, ONa, OK, OH oder ONH₄ , bevorzugt für ONa, OK, OH oder ONH₄ steht. In der bevorzugten Form stehen die Gruppen in folgendem Molverhältnis zueinander: ONa : OK : OH : ONH₄ = 0 - 3 : 0 - 3 : 0 - 2 : 0,1 - 4, bevorzugt 0 - 2,5 : 0 - 2,5 : 0 - 1,5 : 0,5 - 3,5, besonders bevorzugt 0 - 2,2 : 0 - 2,2 : 0 - 1,2 : 0,8 - 3,2. Für die erfindungsgemäßen Nebenkomponenten ergeben sich die Molverhältnisse entsprechend der Anzahl ihrer Carboxylgruppen.

Bezeichnet man das Iminodibernsteinsäureanion als IDS, so können die erfindungsgemäßen Iminodibernsteinsäureammoniumsalze in der bevorzugten Form auch durch die Formel 6 beschrieben werden:
IDS(NH₄)ₓ(Na)_{y}(K)_{z}(H)ₘ Formel 6
mit x = 0,1 - 4, y = 0 - 3, z = 0 - 3 und m = 0 - 2, bevorzugt x = 0,5 - 3,5, y = 0 - 2,5, z = 0 - 2,5 und m = 0 - 1,5, besonders bevorzugt x = 0,8 - 3,2, y = 0 - 2,2, z = 0 - 2,2 und m = 0 - 1,2 und IDS für den Iminodibernsteinsäurerest steht.

In besonderen Ausführungsformen erhält man die besonders bevorzugten erfindungsgemäßen Zwischenprodukte oder ihre Mischungen:

IDS(NH₄)₃K, IDS(NH₄)₂K₂, IDS(NH₄)K₂H, IDS(NH₄)₂KH und IDS(NH₄)K₃ oder IDS(NH₄)₃Na, IDS(NH₄)₂Na₂, IDS(NH₄)Na₂H, IDS(NH₄)₂NaH und IDS(NH₄)Na₃ oder IDS(NH₄)₃H und IDS(NH₄)₂H₂. Prinzipiell ist auf diese Weise auch das IDS(NH₄)₄ herstellbar. Diese, gemäß der vorliegenden Erfindung herzustellenden Zwischenprodukte der Formel 6, können im Photobereich zur Anwendung kommen. Bevorzugt werden alkalifreie Iminodibernsteinsäureammoniumsalze, besonders bevorzugt IDS(NH₄)₃H, IDS(NH₄)₂H₂ und IDS(NH₄)₄ , eingesetzt.

Erfindungsgemäß besonders bevorzugt für die Umsetzung in der zweiten Stufe sind alkalifreie Iminodibernsteinsäureammoniumsalze der Formel 7.
IDS(NH₄)ₓ(H)_{y} Formel 7
mit x = 2 - 4 und y = 0 - 2, bevorzugt mit x = 2,5 - 3,5 und y = 0,5 - 1,5, besonders bevorzugt mit x = 2,75 - 3,25 und y = 0,75 - 1,25 und IDS der Iminodibernsteinsäurerest. Erfindungsgemäß ganz besonders bevorzugt ist das Iminodibernsteinsäuretriammoniumsalz gemäß Formel 7 mit x = 3 und y = 1.

In der zweiten Stufe des erfindungsgemäßen Verfahrens werden die mit oder ohne Aufarbeitung hergestellten Iminodibernsteinsäureammoniumsalze der Formel 6 oder der Formel 7 mit Metalloxiden, Metallhydroxiden oder anderen Metallsalzen in wässriger Lösung zu den erfindungsgemäßen Iminodibernsteinsäureammoniummetallsalzen der Formel 8
IDS(NH₄)ₓ(Na)_{y}(K)_{z}(Me)ₘ·(NH₃)ₙ Formel 8
umgesetzt, in der IDS für den Iminodibernsteinsäurerest steht und x = 0,1 - 3,9, y = 0 - 3, z = 0 - 3, m = 0,1 - 2 und n = 0 - 6, bevorzugt mit x = 0,1 - 3, y = 0 - 2,5, z = 0 - 2,5, m = 0,4 - 1,8 und n = 0 - 4, besonders bevorzugt mit x = 0,1 - 2, y = 0 - 2,2, z = 0 - 2,2, m = 0,43 - 1,7 und n = 0 - 3 und ganz besonders bevorzugt mit x = 0,5 - 2, y = 0, z = 0 oder 2, m = 0,43 - 1,6 und n = 0 - 2,5 bedeutet und

Me für Metalle der II., III. und IV. Hauptgruppe, der I. bis VIII. Nebengruppe sowie für Metalle der Lanthaniden des Periodensystems steht, die in den Oxidationsstufen 1, 2, 3 oder 4 vorkommen können, bevorzugt in den Oxidationsstufen 2 und 3 vorliegen mit Ausnahme der S,S-Isomeren der Mg und Ca Iminodibernsteinsäureammoniummetallsalze. Me steht bevorzugt für die Metalle Mg, Ca, Sr, Ba, Ti, Zr, Cr, Mo, W, Mn, Fe, Co, Ni, Cu, Ag, Zn, Cd, Al, Sn, Pb, La oder Ce und besonders bevorzugt für die Metalle Mg, Ca, Ti, Zr, Cr, Mo, Mn, Fe, Co, Ni, Cu, Zn, Cd, Al, Pb und Ce. In ganz besonders bevorzugter Weise steht Me für die Metalle Ti, Zr, Cr, Mo, Co, Ni, Cd, Al, Pb und Ce.

Der in der Formel 8 mit (NH₃)ₙ dargestellte Ammoniak kann in freier Form, in komplex-gebundener Form oder als Ammoniumsalz mit den Anionen vorliegen, die bei der Verwendung von Metallsalzen in die Produktlösung mit eingebracht werden.

In einer praktischen Ausführungsform werden die in der ersten Stufe hergestellten Iminodibernsteinsäureammoniumsalzlösungen mit Metalloxiden, Metallhydroxiden oder anderen Metallsalzen bzw. deren Mischungen sowie mit weiterem Wasser versetzt und gegebenenfalls unter Abdestillation von Ammoniakwasser in die erfindungsgemäßen Iminodibernsteinsäureammoniummetallsalze überführt. Die Zugabe kann kontinuierlich oder diskontinuierlich in Portionen bei Temperaturen von 10 - 140°C, bevorzugt bei 15 - 120°C und besonders bevorzugt bei 20 - 100°C innerhalb von 0,1 - 100 h, bevorzugt 0,2 - 50 h, besonders bevorzugt innerhalb von 0,5 - 25 h erfolgen. Die Geschwindigkeit der Zugabe wird durch die Rührbarkeit des Reaktionsgemisches begrenzt.

Die Metalloxide, Metallhydroxide oder anderen Metallsalze oder ihre Mischungen können als Feststoff, Suspension, Dispersion, Slurry oder Lösung zugegeben werden. Dabei können Hilfsstoffe wie z.B. Polycarbonsäuren oder Polysulfonsäuren, die als Dispergiermittel oder Thresholdinhibitor fungieren können, verwendet werden. Bevorzugt sind umweltfreundliche, biologisch abbaubare Polycarbonsäuren wie z.B. Polyaminosäuren, besonders bevorzugt sind Polyasparaginsäuren. Alkyl- und Arylamine können in der zweiten Reaktionsstufe zur zusätzlichen Komplexierung der Metallionen als Hilfsstoffe hinzugesetzt werden. Bevorzugt werden Alkylamine, wobei die Aminogruppe einfach, zweifach oder dreifach substituiert sein kann, eingesetzt. Als Alkylamine können Mono-, Di, Tri- und Polyamine verwendet werden. Beispiele sind Ethanolamin, Diethanolamin, Triethanolamin, Ethylendiamin oder Aminosacharide. Bevorzugt werden biologisch abbaubare Amine eingesetzt. Als weitere Hilfsstoffe können Alkohole, z.B. Alkanole, Diole, Triole oder Polyole eingesetzt werden. Bevorzugt können biologisch abbaubare Alkohole, z. B. Ethylenglycol, eingesetzt werden.

Erfindungsgemäß in Stufe 2 des Verfahrens einzusetzende Metalloxide sind z.B. MgO, CaO, TiO, Ti₂O₃, TiO₂, ZrO₂, Chrom(III)-oxid, MnO, Mn₂O₃, MnO₂, Eisen(II)-oxid, Eisen(III)-oxid, Kobalt(II)-oxid, Kupfer(II)-oxid, Zinkoxid, Cadmiumoxid und Cer(IV)-oxid. Als Metallhydroxide können z.B. Mg(OH)₂, Ca(OH)₂, Zr(OH)₄, frisch gefälltes Eisenoxid-hydroxid, Kobalt(II)-hydroxid, Nickel(II)-hydroxid, Kupfer(II)-hydroxidcarbonat, Kupfer(II)-hydroxid, Kupfer(II)-hydroxidphosphat, Zinkhydroxidcarbonat, Cadmiumhydroxid, Aluminiumhydroxid, Lanthan(III)-hydroxid und Cer(IV)-hydroxid verwendet werden. Die anderen Metallsalze sind Salze anorganischer und organischer Säuren wie z.B. Halogenide, Sulfate, Nitrate, Phosphate, Chlorate, Perchlorate, Carbonate, Acetate, Formiate, Gluconate, Oxalate, Sulfonate und Citrate. Bevorzugt werden MgCl₂, CaCl₂, Zirkonacetat, Zirkon(IV)-acetat-hydroxid, Zirkon(IV)citrat, Zirkon(IV)-hydrogenphosphat, Chrom(II)-acetat Hydrat, Chrom(III)-acetat-hydroxid, Chrom(III)-chlorid, Chrom(III)-nitrat, Chrom(III)-sulfat, Mangan(II)-acetat, Mangan(II)-chlorid, Mangan(II)-nitrat, Mangan(II)-sulfat, Eisen(II)-acetat, Eisen(II)-chlorid, Eisen(III)-chlorid, Eisen(III)-citrat, Eisen(III)-pyrophosphat Hydrat, Eisen(III)-nitrat, Eisen(III)-oxalat, Eisen(II)-D-gluconat, Eisen(III)-perchlorat, Eisen(III)-phosphat, Eisen(II)-sulfat, Eisen(III)-sulfat, Eisen(III)-p-toluolsulfonat, Kobalt(II)-acetat, Kobalt(II)-carbonat, Kobalt(II)-chlorid, Kobalt(II)-nitrat, Kobalt(II)-oxalat, Kobalt(II)-sulfat, Nickel(II)-acetat, Nickel(II)-chlorid, Nickel(II)-nitrat, Nickel(II)-oxalat, Nickel(II)-perchlorat, Nickel(II)-sulfat, Kupfer(I)-acetat, Kupfer(II)-acetat, Kupfer(I)-chlorid, Kupfer(II)-chlorid, Kupfer(II)-nitrat, Kupfer(II)-perchlorat, Kupfer(II)-sulfat, Silberacetat, Silbernitrat, Silberperchlorat, Zinkacetat, Zinkchlorid, Zinkcitrat, Zinknitrat, Zinkperchlorat, Zinksulfat, Cadmiumacetat, Cadmiumcarbonat, Cadmiumchlorid, Cadmiumnitrat, Cadmiumperchlorat, Cadmiumsulfat, Aluminiumacetat basisch, Aluminiumchlorid, Aluminiumnitrat, Aluminiumsulfat, Zinn(II)-acetat, Zinn(II)-chlorid, Zinn(II)-methansulfonat, Zinn(II)-sulfat, Zinn(IV)-sulfat, Blei(II)-acetat basisch, Blei(II)-carbonat basisch, Blei(II)-nitrat, Blei(II)-perchlorat, Lanthan(III)-acetat, Lanthan(III)-carbonat, Lanthan(III)-chlorid, Lanthan(III)-nitrat, Lanthan(III)-sulfat, Lanthan(III)-perchlorat, Lanthan(III)-triflat, Cer(IV)-ammoniumsulfat, Cer(IV)-ammoniumnitrat, Cer(IV)-perchlorat und Cer(IV)-sulfat verwendet. Alle genannten Verbindungen können einzeln oder als Mischungen eingesetzt werden.

Nach der Zugabe der Oxide, Hydroxide oder Salze wird bei Temperaturen von 10 - 140°C, bevorzugt bei 15 - 120°C und besonders bevorzugt bei 20 - 100°C innerhalb von 0,1 - 100 h, bevorzugt 0,2 - 50 h, besonders bevorzugt innerhalb von 0,5 - 25 h nachgerührt. Während der Zugabe oder der Nachrührzeit kann Ammoniakwasser abdestilliert werden. Durch Zugabe von Wasser oder Ammoniakwasser wird eine geeignete Konzentration von z.B. über 10 Gew.-%, bevorzugt über 20 Gew.-%, besonders bevorzugt über 30 Gew.-% bzw. ein geeigneter pH-Wert eingestellt. Sofern ungelöste Bestandteile verblieben sind können diese abfiltriert werden. Bevorzugt werden Metalloxide, Metallhydroxide oder andere Metallsalze oder deren Mischungen eingesetzt, bei denen nur eine Klärfiltration in diesem Verfahrensschritt nötig ist. Die erfindungsgemäß hergestellten Lösungen, die auch freien Ammoniak enthalten können, können anschließend getrocknet, beispielsweise sprühgetrocknet, werden. Dabei entstehen Produkte mit einem Feststoffgehalt von über 75 Gew.-%, bevorzugt über 80 Gew.-%, besonders bevorzugt über 85 Gew.-%.

Gegenstand der vorliegenden Erfindung sind aber auch die Iminodibernsteinsäureammoniummetallsalze der Formel 8
IDS (NH₄)ₓ (Na)_{y} (K)_{z}(Me)ₘ . (NH₃)ₙ Formel 8
in der IDS für den Iminodibernsteinsäurerest steht und x = 0,1 - 3,9, y = 0 - 3, z = 0 - 3, m = 0,1 - 2 und n = 0 - 6, bevorzugt mit x = 0,1 - 3, y = 0 - 2,5, z = 0 - 2,5, m = 0,4 - 1,8 und n = 0 - 4, besonders bevorzugt mit x = 0,1 - 2, y = 0 - 2,2, z = 0 - 2,2, m = 0,43 - 1,7 und n = 0 - 3 und ganz besonders bevorzugt mit x = 0,5 - 2, y = 0, z = 0 oder 2, m = 0,43 - 1,6 und n = 0 - 2,5 bedeutet und

Me für Metalle der II., III. und IV. Hauptgruppe, der I. bis VIII. Nebengruppe sowie für Metalle der Lanthaniden des Periodensystems steht, die in den Oxidationsstufen 1, 2, 3 oder 4 vorkommen können, bevorzugt in den Oxidationsstufen 2 und 3 vorliegen mit Ausnahme der S,S-Isomeren der Mg und Ca Iminodibernsteinsäureammoniummetallsalze.

Die Erfindung betrifft bevorzugt Verbindungen der Formel 8 worin x, y, z, m und n die oben genannten Bedeutungen haben und Me für die Metalle Mg, Ca, Sr, Ba, Ti, Zr, Cr, Mo, W, Mn, Fe, Co, Ni, Cu, Ag, Zn, Cd, Al, Sn, Pb, La oder Ce und besonders bevorzugt für die Metalle Mg, Ca, Ti, Zr, Cr, Mo, Mn, Fe, Co, Ni, Cu, Zn, Cd, Al, Pb und Ce steht mit Ausnahme der S,S-Isomeren der Mg und Ca Iminodibernsteinsäureammoniummetallsalze. In ganz besonders bevorzugter Weise steht Me für die Metalle Ti, Zr, Cr, Mo, Ni, Cd, Al und Pb.

Auch die in der zweiten Stufe erfindungsgemäß hergestellten Iminodibernsteinsäureammoniummetallsalzlösungen oder Feststoffe zeigen folgende Zusammensetzung: Iminodibernsteinsäureammoniummetallsalze (Formel 8) als Gemisch der Stereoisomere (S,S-IDS, R,R-IDS und R,S-IDS) in Ausbeuten von >65 %, bevorzugt >70 %, besonders bevorzugt >74 % der theoretischen Ausbeute. Die Summe aller Nebenkomponenten und ihrer Salze liegen in Mengen von <35 %, bevorzugt <30 %, besonders bevorzugt <26 % der theoretischen Mengen vor, wobei Maleinsäure und ihre Salze mit <7 %, bevorzugt <5 %, besonders bevorzugt <2 % der theoretischen Menge, Fumarsäure und ihre Salze mit <20 %, bevorzugt <15 %, besonders bevorzugt <10 % der theoretischen Menge, Äpfelsäure als Gemisch der Stereoisomere ( R- und S-Äpfelsäure ) und ihre Salze mit <7 %, bevorzugt <5 %, besonders bevorzugt <3 % der theoretischen Menge und Asparaginsäure als Gemisch der Stereoisomere ( Rund S-Asparaginsäure ) und ihre Salze mit <25 %, bevorzugt <20 %, besonders bevorzugt <15 % der theoretischen Menge vorliegen.

Die Produkte sind überraschenderweise auch biologisch abbaubar und damit von hohem Vorteil für die Umwelt. Im Zahn Wellens Test, dem OECD 302 B, wurde das IDS K₂Zn_{0,5}(NH₄)_{0,5}-Salz (Beispiel 23) bereits nach 14 d zu 98 % und das IDS K₂Cu_{0,43}(NH₄)_{0,58}-Salz (Beispiel 24) nach 28 d zu 99 % abgebaut (d = Tage).

Die beschriebenen Produkte können in allen Anwendungsgebieten eingesetzt werden, in denen die Erhöhung der Verfügbarkeit von Spurenelementen bzw. Metallionen benötigt wird, z.B. in der Landwirtschaft oder Gärtnerei als Spurennährstoffdünger oder Schneckenkorn oder in der Keramikindustrie zur Färbung von Oberflächen.

Sehr viele Metallionen bilden in Anwendungsgebieten, in denen Wasser ein grundlegendes Medium darstellt, unlösliche Metallsalze und werden so der eigentlichen Anwendung entzogen. Beim Einsatz von einfachen anorganischen oder organischen in Wasser löslichen Salzen würden auf diese Weise die Metallionen ihren bestimmungsgemäßen Wirkort nicht erreichen. Beispielhaft wären die Spurenelemente für die Pflanzen nicht nutzbar und die farbgebenden Metallionen würden nicht tief genug in keramisches Material eindringen können.

Mit der Hilfe von Komplexiermitteln gelingt es nun die Bildung von unlöslichen Metallsalzen zu verhindern und die benötigten Metallionen zu ihrem Wirkort zu bringen. Bei den bisher eingesetzten Verbindungen handelt es sich überwiegend um biologisch nicht oder schwer abbaubare starke Komplexiermittel, die einerseits zwar die Bildung von unlöslichen Metallsalzen nahezu vollständig verhindern, andererseits aber auch die Verfügbarkeit z.B. der Spurenelemente bedingt durch die starke Komplexierung begrenzen.

Durch die Verwendung der erfindungsgemäßen Iminodibernsteinsäureammoniummetallsalze wird nun die Verfügbarkeit von Metallionen, beispielsweise der Spurenelemente Mg, Ca, Mn, Cu, Fe und Zn als Spurennährstoffdünger erhöht und auch der Einsatz im Keramikbereich als Metallioncarrier bei der Herstellung von Keramiken oder Lasuren eröffnet. Darüber hinaus sind die neuen Salze biologisch abbaubar und damit umweltfreundlicher als die bisherigen Produkte. Bedingt durch den Abbau kann sogar das Anion in biologischen Anwendungen zusätzlich als Nahrungsquelle dienen. Die intermediären Abbauprodukte sind Asparaginsäure und Fumarsäure, zwei in der Natur vorkommende Verbindungen, was ein zusätzlicher Vorteil ist.

Als besonders bevorzugte Spurennährstoffdünger auf Basis der Iminodibernsteinsäureammoniumsalze eignen sich natriumarme Mischsalze.

Im letzten Jahrzehnt hat die Einfärbung von Fliesenscherben aus Gres Porcellanato mit konzentrierten wässrigen Lösungen von Schwermetallen an Bedeutung gewonnen. Dabei werden die Scherben oberflächlich mit der Metallsalzlösung besprüht, bestrichen oder der ganze Körper in die Lösung getaucht, anschließend getrocknet und gebrannt. Durch den Brand wandeln sich die Metallkomplexe in die entsprechenden gefärbten Oxide um.

Für das Verfahren ist die Herstellung möglichst konzentrierter, sulfat-, nitrat- und chloridfreier Lösungen wichtig (keine korrosiven Abgase während des Brandes). Eine weitere Bedingung ist die gute Penetrationsfähigkeit der Metallsalzlösungen die diese insbesondere bei Gres-Porcellanato-Fliesen zeigen, da diese nach dem Brand geschliffen werden (Marmor-Effekt). Die erfindungsgemäßen Iminodibernsteinsäuremetallsalze stellen für diesen Zweck in Lösung ausreichende Mengen Schwermetall bis in die tieferen Schichten dieser Fliesen zur Verfügung.

Die erfindungsgemäßen Iminodibernsteinsäureammoniumsalze der Formeln 6 und 7, insbesondere die Iminodibernsteinsäuretriammoniumsalze erweisen sich überraschend als geradezu ideal für diese Anwendung, da farbgebende Schwermetalle (Cobalt, Nickel, Eisen, Kupfer, Chrom) gut komplexiert und in hoher Konzentration in Lösung gehalten werden können, ohne dass Chlorid, Nitrat usw. als Gegenion vorhanden sein muss.

### Beispiele

### Herstellung von Iminodibernsteinsäureammoniumsalzen

### Beispiel 1

### MSA:NH₃:H₂O-Molverhältnis = 2:4:9,90°C, 30 h

1782 g = 99 Mol Wasser werden vorgelegt und auf 80°C erwärmt. 2157,3 g = 22 Mol Maleinsäureanhydrid werden unter Rühren und Kühlen bei 80-100°C zugegeben. Nachdem das Maleinsäureanhydrid in Lösung gegangen ist, werden noch ca. 0,5 h nachgeführt. Anschließend werden 748 g = 44 Mol Ammoniak bei >90°C eingeleitet. Nach der beendeten Ammoniakeinleitung wird das Reaktionsgemisch bei 90°C über 30 h lang gerührt, mit 1312,7 g = 72,93 mol Wasser verdünnt und auf Raumtemperatur gekühlt. Nach einer Klärfiltration, die gegebenenfalls durchzuführen ist, werden 6000 g Produktlösung mit folgenden Ausbeuten erhalten: 80,0 % d.Th. Iminodibernsteinsäureammoniumsalz, 14,6 % d.Th. Asparaginsäureammoniumsalz, 2,5 % d.Th. Fumarsäureammoniumsalz, 0,6 % d.Th. Äpfelsäureammoniumsalz und 0,4 % d.Th. Maleinsäureammoniumsalz. Der Feststoffgehalt (= Σ Ammoniumsalze) beträgt 55 Gew.-%. Die klare hellgelbe Lösung weist eine Dichte von 1,242 kg/Liter und einen pH-Wert von 7,3 auf.

### Beispiel 2

### MSA:NH₃:H₂O-Molverhältnis = 2:4:9,90°C, 24 h

Durchführung und Mengen entsprechen Beispiel 1. Die Reaktionszeit beträgt 24 h. Folgende Ausbeuten werden erhalten: 77,9 % d.Th. Iminodibernsteinsäureammoniumsalz, 12,8 % d.Th. Asparaginsäureammoniumsalz, 3,0 % d.Th. Fumarsäureammoniumsalz, 1,0 % d.Th. Äpfelsäureammoniumsalz und 3,4 % d.Th. Maleinsäureammoniumsalz.

### Beispiel 3

### MSA:NH₃:H₂O-Molverhältsnis = 2:4:9,90°C, 36 h

Durchführung und Mengen entsprechen Beispiel 1. Die Reaktionszeit beträgt 36 h. Folgende Ausbeuten werden erhalten: 80,1 % d.Th. Iminodibernsteinsäureammoniumsalz, 15,0 % d.Th.Asparaginsäureammoniumsalz, 3,3 % d.Th. Fumarsäureammoniumsalz, 1,1 % d.Th. Äpfelsäureammoniumsalz und 0,6 % d.Th. Maleinsäureammoniumsalz. Der Feststoffgehalt (= Σ Ammoniumsalze) beträgt 55 Gew.-%. Die klare hellgelbe Lösung weist eine Dichte von 1,243 kg/Liter und einen pH-Wert von 7,03 auf.

### Beispiel 4

### MSA:NH₃:H₂O-Molverhältnis = 2:4:10, 80°C, 96 h

Wasser, Maleinsäureanhydrid und Ammoniak werden im Molverhältnis 10:2:4 wie in Beispiel 1 zusammengegeben. Bei 80°C beträgt die Reaktionszeit 96 h. Nach Verdünnung und gegebenenfalls Klärfiltration wird Iminodibernsteinsäureammoniumsalz mit einer Ausbeute von 82,5 % d.Th. erhalten.

### Beispiel 5

### MSA:NH₃:H₂O-Molverhältnis = 2:4:10, 100°C, 9 h

Wasser, Maleinsäureanhydrid und Ammoniak werden im Molverhältnis 10:2:4 wie in Beispiel 1 zusammengegeben. Bei 100°C beträgt die Reaktionszeit 9 h. Nach Verdünnung und gegebenenfalls Klärfiltration wird Iminodibernsteinsäureammoniumsalz mit einer Ausbeute von 77,8 % d.Th. erhalten.

### Beispiel 6

### MSA:NH₃:H₂O-Molverhältnis = 2:4:10,110°C, 4 h

Wasser, Maleinsäureanhydrid und Ammoniak werden im Molverhältnis 10:2:4 wie in Beispiel 1 zusammengegeben. Bei 110°C beträgt die Reaktionszeit 4 h. Nach Verdünnen und gegebenenfalls Klärfiltration wird Iminodibernsteinsäureammoniumsalz mit einer Ausbeute von 76,9 % d.Th. erhalten.

### Beispiel 7

### MSA:NH₃:H₂O-Molverhältnis = 2:4:10, 120°C, 2 h

Wasser, Maleinsäureanhydrid und Ammoniak werden im Molverhältnis 10:2:4 wie in Beispiel 1 zusammengegeben. Bei 120°C beträgt die Reaktionszeit 2 h. Nach Verdünnung und gegebenenfalls Klärfiltration wird Iminodibernsteinsäureammoniumsalz mit einer Ausbeute von 67,9 % d.Th. erhalten.

### Beispiel 8

### MSA:NH₃:H₂O-Molverhältnis = 2:4:8, 80°C, 72 h

Wasser, Maleinsäureanhydrid und Ammoniak werden im Molverhältnis 8:2:4 wie in Beispiel 1 zusammengegeben. Bei 80°C beträgt die Reaktionszeit 72 h. Nach Verdünnung und gegebenenfalls Klärfiltration wird Iminodibernsteinsäureammoniumsalz mit einer Ausbeute von 82,7 % d.Th. erhalten.

### Beispiel 9

### MSA:NH₃:H₂O-Molverhältnis = 2:4:6,80°C, 60 h

Wasser, Maleinsäureanhydrid und Ammoniak werden im Molverhältnis 6:2:4 wie in Beispiel 1 zusammengegeben. Bei 80°C beträgt die Reaktionszeit 60 h. Nach Verdünnung und gegebenenfalls Klärfiltration wird Iminodibernsteinsaureammoniumsalz mit einer Ausbeute von 82,2 % d.Th. erhalten.

### Beispiel 10

### MSA:NH₃:H₂O-Molverhältnis = 2:4:6, 120°C, 1 h

Wasser, Maleinsäureanhydrid und Ammoniak werden im Molverhältnis 6:2:4 wie in Beispiel 1 zusammengegeben. Bei 120°C beträgt die Reaktionszeit 1 h. Nach Verdünnung und gegebenenfalls Klärfiltration wird Iminodibernsteinsäureammoniumsalz mit einer Ausbeute von 71,9 % d.Th. erhalten.

### Beispiel 11

### MSA:NH₃:H₂O-Molverhältnis = 2:6:10, 80°C, 54 h

Wasser, Maleinsäureanhydrid und Ammoniak werden im Molverhältnis 10:2:6 wie in Beispiel 1 zusammengegeben. Bei 80°C beträgt die Reaktionszeit 54 h. Nach Verdünnung und gegebenenfalls Klärfiltration wird Iminodibernsteinsäureammoniumsalz mit einer Ausbeute von 79,0 % d.Th. erhalten.

### Beispiel 12

### MSA:NH₃:H₂O-Molverhältnis = 2:6:10,110°C, 2 h

Wasser, Maleinsäureanhydrid und Ammoniak werden im Molverhältnis 10:2:6 wie in Beispiel 1 zusammengegeben. Bei 110°C beträgt die Reaktionszeit 2 h. Nach Verdünnung und gegebenenfalls Klärfiltration wird Iminodibernsteinsäureammoniumsalz mit einer Ausbeute von 69,1 % d.Th. erhalten.

### Beispiel 13

### MSA:NH₃:H₂O-Molverhältnis = 2:6:8, 80°C, 42 h

Wasser, Maleinsäureanhydrid und Ammoniak werden im Molverhältnis 8:2:6 wie in Beispiel 1 zusammengegeben. Bei 80°C beträgt die Reaktionszeit 42 h. Nach Verdünnung und gegebenenfalls Klärfiltration wird Iminodibernsteinsäureammoniumsalz mit einer Ausbeute von 77,3 % d.Th. erhalten.

### Beispiel 14

### MSA:NH₃:H₂O-Molverhältnis = 2:6:6, 120°C, 1 h.

Wasser, Maleinsäureanhydrid und Ammoniak werden im Molverhältnis 6:2:6 wie in Beispiel 1 zusammengegeben. Bei 120°C beträgt die Reaktionszeit 1 h. Nach Verdünnung und gegebenenfalls Klärfiltration wird Iminodibernsteinsäureammoniumsalz mit einer Ausbeute von 61,0 % d.Th. erhalten.

### Beispiel 15

### MSA:KOH:NH₃:H₂O-Molverhältnis = 2:2:2,3:10, 110°C, 8 h.

679,8 g = 37,73 Mol Wasser werden vorgelegt und auf 80°C erwärmt. Anschließend werden 1961,2 g = 20 Mol MSA als Schmelze und 2244,4 g = 20 Mol KOH als 50%ige Lösung bei 95 - 105°C innerhalb von 4 h simultan zudosiert. Nach der Zugabe von 391,7 g = 23 Mol Ammoniak, die bei 90 - 105°C erfolgt, wird das Reaktionsgemisch 8 h bei 110°C unter Druck gerührt. Man erhält 5277,1 g Reaktionsgemisch, die direkt zur Bildung der Iminodibernsteinsäureammoniummetallsalze eingesetzt werden können.

### Beispiel 16

Zu den 5277,1 g Reaktionsgemisch aus Beispiel 15 werden 200 g Wasser gegeben und bei 95 - 112°C 230 g Ammoniakwasser abdestilliert. Anschließend wird mit Ammoniakwasser auf pH 7,5 eingestellt und mit Wasser auf 5863 g = 4248,6 ml aufgefüllt. Nach einer Klärfiltration bei 20 - 40°C erhält man eine Iminodibernsteinsäure K₂NH₄-Salzlösung mit einem Feststoffgehalt von 58,4 Gew.-%. Folgende Ausbeuten wurden gefunden: 79,7 % d.Th. IDS K₂NH₄-Salz, 14,5 % d.Th. Asparaginsäurekaliumammoniumsalz, 4,4 % d.Th. Fumarsäurekaliumammoniumsalz, 0,7 % d.Th. Maleinsäurekaliumammoniumsalz und 0,2 % d.Th. Äpfelsäurekaliumammoniumsalz.

### Umsetzung zu Iminodibernsteinsäureammoniummetallsalzen

### Beispiel 17

### Herstellung von Iminodibernsteinsäure Zn NH₄-Salz

545,5 g Produktlösung aus Beispiel 1 werden vorgelegt und auf 60°C erwärmt. 81,4 g ZnO 100 %ig = 1 Mol Zinkoxid werden in 10 Portionen zu je 8,14 g hinzugegeben. Nach insgesamt 2,5 h ist das gesamte Zinkoxid in Lösung gegangen. Man erhält 626,9 g einer klaren hellgelben auch bei 1°C lagerstabilen Lösung. Folgende Werte werden bestimmt: pH = 7,8 bei 23°C.

| | Berechnet | Gefunden |
|---|---|---|
| C | 15,3 % | 15,5 % |
| N | 8,93 % | 9,0 % |
| Zn | 10,4 % | 9,9 % |

Dies entspricht einer Verbindung der Formel IDS (NH₄)₂ Na₀ K₀ Zn₁ · (NH₃)₁. Die Trocknung dieser Lösung bei 40 - 80°C ergab einen Feststoff mit folgenden Werten: C_{gef}. = 22,6 Gew.-%, N_{gef}. = 12,4 Gew.-%. Das entspricht einer Verbindung der Formel IDS (NH₄)₂ Na₀ K₀ Zn₁ · (NH₃)_{0,76}.

### Beispiel 18

### Herstellung von Iminodibernsteinsäure Cu NH₄-Salz

545,5 g Produktlösung aus Beispiel 1 werden vorgelegt und auf 35°C erwärmt. 113,5 g Cu(OH)₂ 86 %ig = 1 Mol Kupferhydroxid werden in 10 Portionen zu 11,35 g bei 35-50°C hinzugegeben. Nach 1 h ist das gesamte Kupferhydroxid in Lösung gegangen. Man erhält 659 g einer tiefblauen bei 1°C lagerstabilen Lösung. Folgende Werte werden bestimmt: pH = 7,1 bei 23°C.

| | Berechnet | Gefunden |
|---|---|---|
| C | 14,6 % | 14,5 % |
| N | 8,5 % | 8,3 % |
| Cu | 9,6 % | 9,8 % |

Dies entspricht einer Verbindung der Formel IDS (NH₄)₂ Na₀ K₀ Cu₁ · (NH₃)₁. Die Lösung kann bei 40 - 80°C getrocknet werden. Man erhält einen Feststoff mit folgenden Werten: C_{gef}. = 22,5 Gew.-%, N_{gef}. = 12,5 Gew.-%. Das entspricht einer Verbindung der Formel IDS (NH₄)₂ Na₀ K₀ Cu₁ · (NH₃)_{0,8}.

### Beispiel 19

### Herstellung von Iminodibernsteinsäure Mg NH₄-Salz

545,5 g Produktlösung aus Beispiel 1 werden vorgelegt und auf 40°C erwärmt. 61,4 g Mg(OH)₂ 95 %ig = 1 Mol Magnesiumhydroxid werden in 10 Portionen zu 6,14 g bei 40-80°C hinzugegeben. Nach 4 h ist das Magnesiumhydroxid unter leichter Ammoniakentwicklung fast vollständig in Lösung gegangen. Die leicht trübe Lösung wird noch ca. 4 h bei 80°C und 1 h bei 90°C nachgerührt. Nach einer Klärfiltration erhält man 590 g einer fast farblosen bei 20°C lagerstabilen Lösung. Folgende Werte werden bestimmt: pH = 9,2 bei 24°C.

| | Berechnet | Gefunden |
|---|---|---|
| C | 15,8 % | 16,4 % |
| N | 9,2 % | 7,9 % |
| Mg | 4,0 % | 3,9 % |

Dies entspricht einer Verbindung der Formel IDS (NH₄)₂ Na₀ K₀ Mg₁ · (NH₃)₁. Die Lösung kann bei 40 - 80°C getrocknet werden. Man erhält einen Feststoff mit folgenden Werten: C_{gef}. = 26,4 Gew.-%, N_{gef}. = 10,7 Gew.-%. Das entspricht einer Verbindung der Formel IDS (NH₄)_{1,78} Na₀ K₀ Mg₁ · (NH₃)₀.

### Beispiel 20

### Herstellung von Iminodibernsteinsäure Ca NH₄-Salz

545,5 g Produktlösung aus Beispiel 1 werden vorgelegt und auf 60°C erwärmt. 77,2 g Ca(OH)₂ 96 %ig = 1 Mol Calciumhydroxid werden in 10 Portionen zu 7,72 g bei ca. 60-65°C hinzugegeben. Nach 8 Portionen und ca. 2,5 h liegt eine fast klare Lösung vor. Nach weiteren 2 Portionen und ca. 3 h wird eine trübe Lösung erhalten.

Nach einer Klärfiltration erhält man 605 g einer fast farblosen bei 20°C lagerstabilen Lösung. Folgende Werte werden bestimmt: pH = 9,7 bei 24°C.

| | Berechnet | Gefunden |
|---|---|---|
| C | 15,4 % | 15,8 % |
| N | 9,0 % | 8,1 % |
| Ca | 6,4 % | 6,2 % |

Dies entspricht einer Verbindung der Formel IDS (NH₄)₂ Na₀ K₀ Ca₁ · (NH₃)₁. Die Lösung kann bei 40 - 80°C getrocknet werden. Man erhält einen Feststoff mit folgenden Werten: C_{gef}. = 26,6 Gew.-%, N_{gef}. = 10,0 Gew.-%. Das entspricht einer Verbindung der Formel IDS (NH₄)_{1,58} Na₀ K₀ Ca₁ · (NH₃)₀.

### Beispiel 21

### Herstellung von Iminodibernsteinsäure Mn NH₄-Salz

545,5 g Produktlösung aus Beispiel 1 werden vorgelegt und auf 60°C erwärmt. 258 g (CH₃Co₂)₃ Mn · 2H₂O 95 %ig = 1 Mol Mangan(III)-acetat Dihydrat werden in 2 Portionen zu 129 g bei 60°C hinzugegeben. Nach ca. 3 h ist das Mangan(III)-acetat Dihydrat in Lösung gegangen. Nach einer Klärfiltration erhält man 785 g einer klaren bei 20°C lagerstabilen Lösung. Folgende Werte werden bestimmt: pH = 5,5 bei 24°C.

| | Berechnet | Gefunden |
|---|---|---|
| C | 18,3 % | 18,5 % |
| N | 7,0 % | 7,0 % |
| Mn | 6,8 % | 6,7 % |

Dies entspricht einer Verbindung der Formel IDS (NH₄)₂ Na₀ K₀ Mn₁ · (NH₃)₁. Die Lösung kann bei 40 - 80°C getrocknet werden. Man erhält einen Feststoff mit folgenden Werten: C_{gef}. = 25,4 Gew.-%, N_{gef}. = 9,9 Gew.-%. Das entspricht einer Verbindung der Formel IDS (NH₄)₂ Na₀ K₀ Mn₁ · (NH₃)₁. Der überschüssige Ammoniak liegt in diesem Fall als Ammoniumacetat vor.

### Beispiel 22

### Herstellung von Iminodibernsteinsäure Zn NH₄-Salz

545,5 g Produktlösung aus Beispiel 1 werden vorgelegt und auf 60°C erwärmt. 130,24 g ZnO = 1,6 Mol Zinkoxid werden in 16 Portionen zu 8,14 g bei 60 - 70°C hinzugegeben. Nach ca. 4,5 h ist das Zinkoxid in Lösung gegangen, nachdem kurzzeitig die Temperatur auf 80°C erhöht worden war. Nach einer Klärfiltration erhält man 663 g einer klaren bei 20°C lagerstabilen Lösung. Folgende Werte werden bestimmt:

| | Berechnet | Gefunden |
|---|---|---|
| C | 14,2 % | 14,6 % |
| N | 8,3 % | 8,3 % |
| Zn | 15,5 % | 15,0 % |

Dies entspricht einer Verbindung der Formel IDS (NH₄)_{0,8} Na₀ K₀ Zn_{1,6} · (NH₃)_{2,2}. Die Lösung kann bei 40 - 80°C getrocknet werden. Man erhält einen Feststoff mit folgenden Werten: C_{gef}. = 21,3 Gew.-%, N_{gef}. = 11,9 Gew.-%. Das entspricht einer Verbindung der Formel IDS (NH₄)_{0,8} Na₀ K₀ Zn_{1,6} · (NH₃)_{2,03}.

### Beispiel 23

### Herstellung von IDS (NH₄)_{0,5} Na₀ K₂ Zn_{0,5} · (NH₃)₀-Salz

527,7 g = 1 Mol Iminodibernsteinsäure K₂(NH₄)_{1,3}-Rohprodukt aus Beispiel 15 werden vorgelegt und auf 50°C erwärmt. 40,7 g = 0,5 Mol Zinkoxid werden in Portionen hinzugegeben. Aus der trüben, gelblichen Lösung, die das IDS (NH₄)₁ Na₀ K₂ Zn_{0,5} · (NH₃)_{0,3} enthält, werden bei 80 - 115°C insgesamt 500 g Ammoniakwasser abdestilliert, das 13,6 g Ammoniak enthält. Während der Destillation wird Wasser in entsprechender Menge wieder hinzugegeben. Nach der Destillation wird mit Wasser auf 595,2 g aufgefüllt. Nach einer Klärfiltration erhält man eine klare, bei 1°C über mehrere Wochen lagerstabile Lösung.

### Beispiel 24

### Herstellung von IDS (NH₄)_{0,58} Na₀ K₂ Cu_{0,43} · (NH₃)₀-Salz

527,7 g = 1 Mol Iminodibernsteinsäure K₂(NH₄)_{1,3}-Rohprodukt aus Beispiel 15 werden vorgelegt und auf 60°C erwärmt. 48,8 g Cu(OH)₂ 86 %ig = 0,43 Mol Kupferhydroxid werden in 5 Portionen zu 9,76 g bei 60°C hinzugegeben. Aus der trüben, blauen Lösung, die das IDS (NH₄)_{1,14} Na₀ K₂ Cu_{0,43} · (NH₃)_{0,16}-Salz enthält, werden bei 80 - 115°C insgesamt 300 g Ammoniakwasser abdestilliert, das 12,17 g Ammoniak enthält. Während der Destillation wird Wasser in entsprechender Menge wieder hinzugegeben. Nach der Destillation wird mit Wasser auf 593,6 g aufgefüllt. Nach einer Klärfiltration erhält man eine klare lagerstabile Lösung.

### Weitere Beispiele

Folgende weitere Produkte sind analog Formel 8 =
IDS(NH₄)ₓ(Na)_{y}(K)_{z}(Me)ₘ (NH₃)ₙ herstellbar:

| Beispiel | Me | x | y | z | m | n |
|---|---|---|---|---|---|---|
| 25 | Al | 1 | 0 | 0 | 1 | 2 |
| 26 | Al | 2,5 | 0 | 0 | 0,5 | 0,5 |
| 27 | Cr | 1 | 0 | 0 | 1 | 2 |
| 28 | Cr | 2,5 | 0 | 0 | 0,5 | 0,5 |
| 29 | Fe^{II} | 2 | 0 | 0 | 1 | 1 |
| 30 | Fe^{II} | 3 | 0 | 0 | 0,5 | 0 |
| 31 | Fe^{III} | 1 | 0 | 0 | 1 | 2 |
| 32 | Fe^{III} | 2,5 | 0 | 0 | 0,5 | 0,5 |
| 33 | Co | 2 | 0 | 0 | 1 | 1 |
| 34 | Co | 3 | 0 | 0 | 0,5 | 0 |
| 35 | Ni | 2 | 0 | 0 | 1 | 1 |
| 36 | Ni | 3 | 0 | 0 | 0,5 | 0 |
| 37 | Ag | 3 | 0 | 0 | 1 | 0 |
| 38 | Ag | 2 | 0 | 0 | 2 | 1 |
| 39 | Cd | 2 | 0 | 0 | 1 | 1 |
| 40 | Cd | 3 | 0 | 0 | 0,5 | 0 |
| 41 | Pb | 2 | 0 | 0 | 1 | 1 |
| 42 | Pb | 3 | 0 | 0 | 0,5 | 0 |
| 43 | La | 2 | 0 | 0 | 1 | 1 |
| 44 | La | 3 | 0 | 0 | 0,5 | 0 |

## Patentansprüche

1. Verfahren zur Herstellung von Iminodibemsteinsäureammoniummetallsalzen, **dadurch gekennzeichnet, dass** in einer ersten Stufe Maleinsäureanhydrid (MSA), Alkalimetallhydroxid und Wasser im Molverhältnis von 2 : 0 - 3 : 5 - 30 vermischt werden und dann Ammoniak im Verhältnis MSA : Ammoniak 2 : 1,5 - 8 hinzudosiert wird um Iminodibemsteinsäureammoniumsalze der Formel 6
IDS(NH₄)ₓ(Na)_{y}(K)_{z}(H)ₘ Formel 6
worin IDS für den Iminodibernsteinsäurerest steht
mit x = 0,1 - 4,y = 0-3, z = 0-3 und m = 0-2
zu erhalten, die in einer zweiten Stufe mit Metalloxiden, Metallhydroxiden oder anderen Metallsalzen oder deren Mischungen zu Iminodibernsteinsäureammoniummetallsalze der Formel 8 umgesetzt werden
IDS (NH₄)ₓ (Na)_{y} (K)_{z} (Me)ₘ • (NH₃)ₙ Formel 8
in der
IDS für den Iminodibemsteinsäurerest steht und
x = 0,1 - 3,9
y = 0 - 3
z = 0 - 3
m = 0,1 - 2
n = 0 - 6
bedeutet und
Me für Metalle der II., III. und IV. Hauptgruppe, der I. bis VIII. Nebengruppe sowie für Metalle der Lanthaniden des Periodensystems steht, die in den Oxidationsstufen 1, 2, 3 oder 4 vorkommen können.

2. Verfahren nach Anspruch 1, **dadurch gekennzeichnet, dass** das Molverhältnis MSA zu Alkalimetallhydroxid in der ersten Stufe 2 : 0 beträgt.

3. Verfahren gemäß der Ansprüche 1 und 2, **dadurch gekennzeichnet, dass** Ammoniak im Verhältnis MSA · Ammoniak 2 : 1,5 - 8 zu den Maleinsäure- bzw. Maleinsäuresalz-haltigen Suspensionen oder Lösungen hinzudosiert wird.

4. Iminodibernsteinsäureammoniummetallsalze der Formel 8
IDS(NH₄)ₓ(Na)_{y}(K)_{z}(Me)ₘ(NH₃)ₙ Formel 8
**dadurch gekennzeichnet, dass**
x = 0,1 - 3,9,
y = 0 - 3,
z = 0 - 3
m = 0,1 - 2
n = 0 - 6
bedeutet, IDS für den Iminodibemsteinsäurerest steht und
Me für Metalle der II., III. und IV. Hauptgruppe, der I. bis VIII. Nebengruppe sowie für Metalle der Lanthaniden des Periodensystems steht, die in den Oxidationsstufen 1, 2, 3 oder 4 vorkommen können, mit Ausnahme der S,S-Isomeren der Mg und Ca Iminodibernsteinsäureammoniummetallsalze.

5. Iminodibernsteinsäureammoniummetallsalze der Formel (8) gemäß Anspruch 4 **dadurch gekennzeichnet, dass** Me für
Mg, Ca, Sr, Ba, Ti, Zr, Cr, Mo, W, Mn, Fe, Co, Ni, Cu, Ag, Zn, Cd, Al, Sn, Pb, La oder Ce steht,
mit Ausnahme der S,S-Isomeren der Ca und Mg Iminodibernsteinsäureammoniummetallsalze.

6. Verwendung der Iminodibernsteinsäureammoniummetallsalze der Formel (8) gemäß Anspruch 4 oder 5 als Spurennährstoffdünger oder Schneckenkom in der Landwirtschaft oder im Gartenbau oder als Metallioncarrier bei der Herstellung von Keramiken oder Lasuren.

## Claims

1. Process for the preparation of iminodisuccinic acid ammonium metal salts, **characterized in that,** in a first stage, maleic anhydride (MA), alkali metal hydroxide and water are mixed in the molar ratio of 2 : 0 - 3 : 5 - 30 and then ammonia is metered in in the ratio MA : ammonia 2 : 1.5 - 8, in order to obtain iminodisuccinic acid ammonium salts of the formula 6
IDA(NH₄)ₓ(Na)_{y}(K)_{z}(H)ₘ Formula 6
in which IDA is the iminodisuccinic acid residue with x = 0.1 - 4, y = 0 - 3, z = 0 - 3 and m = 0 - 2,
which, in a second stage, are reacted with metal oxides, metal hydroxides or other metal salts or their mixtures to give iminodisuccinic acid ammonium metal salts of the formula 8,
IDA(NH₄)ₓ(Na)_{y}(K)_{z}(Me)ₘ•(NH₃)ₙ Formula 8
in which
IDA is the iminodisuccinic acid residue and
x = 0.1 - 3.9
y = 0 - 3
z = 0 - 3
m = 0.1 - 2
n = 0 - 6
and
Me represents metals of the IInd, IIIrd and IVth main groups and of the Ist to VIIIth subgroups as well as metals of the lanthanide series of the Periodic Table, which can occur in the oxidation states 1, 2, 3 or 4.

2. Process according to Claim 1, **characterized in that** the molar ratio of MA to alkali metal hydroxide in the first stage is 2:0.

3. Process according to Claims 1 and 2, **characterized in that** ammonia is metered into the suspensions or solutions comprising maleic acid or maleic acid salt(s) in the MA:ammonia molar ratio of 2:1.5-8.

4. Iminodisuccinic acid ammonium metal salts of the formula 8
IDA(NH₄)ₓ(Na)_{y}(K)_{z}(Me)ₘ(NH₃)ₙ Formula 8
**characterized in that**
x = 0.1 - 3.9
y = 0 - 3
z = 0 - 3
m = 0.1 - 2
n = 0 - 6,
IDA is the iminodisuccinic acid residue and
Me represents metals of the IInd, IIIrd and IVth main groups and of the Ist to VIIIth subgroups as well as metals of the lanthanide series of the Periodic Table, which can occur in the oxidation states 1, 2, 3 or 4, with the exception of the S,S isomers of the Mg and Ca iminodisuccinic acid ammonium metal salts.

5. Iminodisuccinic acid ammonium metal salts of the formula (8) according to Claim 4, **characterized in that** Me represents
Mg, Ca, Sr, Ba, Ti, Zr, Cr, Mo, W, Mn, Fe, Co, Ni, Cu, Ag, Zn, Cd, Al, Sn, Pb, La or Ce,
with the exception of the S,S isomers of the Ca and Mg iminodisuccinic acid ammonium metal salts.

6. Use of the iminodisuccinic acid ammonium metal salts of the formula (8) according to Claim 4 or 5 as trace element fertilizers or slug and snail pellets in agriculture or in horticulture or as metal ion carriers in the preparation of ceramics or glazings.

## Revendications

1. Procédé de fabrication de sels métalliques d'ammonium de l'acide iminodisuccinique, **caractérisé en ce que** lors d'une première étape, de l'anhydride de l'acide maléique (MSA), de l'hydroxyde de métal alcalin et de l'eau sont mélangés en un rapport molaire de 2 : 0 - 3 : 5 - 30, puis de l'ammoniac est ajouté par dosage en un rapport molaire MSA : ammoniac de 2 : 1,5 - 8 afin de former des sels d'ammonium de l'acide iminodisuccinique de formule 6,
IDS(NH₄)ₓ(Nₐ)_{y}(K)_{z}(H)ₘ Formule 6
dans laquelle IDS représente le radical de l'acide iminodisuccinique
avec x = 0,1 - 4, y = 0 - 3, z = 0 - 3 et m = 0 - 2,
qui sont mis en réaction lors d'une seconde étape avec des oxydes métalliques, des hydroxydes métalliques ou d'autres sels métalliques ou leurs mélanges pour former des sels métalliques d'ammonium de l'acide iminodisuccinique de formule 8
IDS(NH₄)ₓ(Na)_{y}(K)_{z}(Me)ₘ•(NH₃)ₙ Formule 8
dans laquelle
IDS représente le radical de l'acide iminodisuccinique
et
x = 0,1 - 3,9
y = 0 - 3
z = 0 - 3
m = 0,1 - 2
n = 0 - 6
et
Me représente des métaux des groupes principaux II, III et IV, des groupes secondaires I à VIII, ainsi que des métaux des lanthanides du tableau périodique, qui peuvent se trouver à l'état d'oxydation 1, 2, 3 ou 4.

2. Procédé selon la revendication 1, **caractérisé en ce que** le rapport molaire entre le MSA et l'hydroxyde de métal alcalin dans la première étape est de 2 : 0.

3. Procédé selon les revendications 1 et 2,
**caractérisé en ce que** l'ammoniac est ajouté par dosage aux suspensions ou solutions contenant de l'acide maléique ou un sel d'acide maléique en un rapport MSA : ammoniac de 2 : 1,5 - 8.

4. Sels métalliques d'ammonium de l'acide iminodisuccinique de formule 8
IDS(NH₄)ₓ(Na)_{y}(K)_{z}(Me)ₘ(NH₃)ₙ Formule 8,
**caractérisés en ce que**
x = 0,1 - 3,9
y = 0 - 3
z = 0 - 3
m = 0,1 - 2
n = 0 - 6,
IDS représente le radical de l'acide iminodisuccinique
et
Me représente des métaux des groupes principaux II, III et IV, des groupes secondaires I à VIII, ainsi que des métaux des lanthanides du tableau périodique, qui peuvent se trouver à l'état d'oxydation 1, 2, 3 ou 4, à l'exception des isomères S,S des sels métalliques d'ammonium de l'acide iminodisuccinique de Mg et Ca.

5. Sels métalliques d'ammonium de l'acide iminodisuccinique de formule 8 selon la revendication 4, **caractérisés en ce que** Me représente
Mg, Ca, Sr, Ba, Ti, Zr, Cr, Mo, W, Mn, Fe, Co, Ni, Cu, Ag, Zn, Cd, Al, Sn, Pb, La ou Ce,
à l'exception des isomères S,S des sels métalliques d'ammonium de l'acide iminodisuccinique de Mg et Ca.

6. Utilisation des sels métalliques d'ammonium de l'acide iminodisuccinique de formule (8) selon la revendication 4 ou 5 en tant qu'engrais à base de micronutriments ou granulés anti-limaces dans l'agriculture ou l'horticulture ou en tant que porteurs d'ions métalliques lors de la fabrication de céramiques ou de glacis.
